# FASCICULE DE BREVET EUROPEEN

(11) **EP 4 346 082 B1**
(45) Date de publication et mention de la délivrance du brevet: **21.08.2024**
(21) Numéro de dépôt: 22315218.2
(22) Date de dépôt: 27.09.2022
(51) Int. Cl.: H02N 2/18, H02N 2/00, A61N 1/378, H10N 30/30

(54) **ENSEMBLE PENDULAIRE À MASSE INERTIELLE MONTÉE SUR UNE LAME PIÉZOÉLECTRIQUE, NOTAMMENT POUR UN RÉCUPÉRATEUR D'ÉNERGIE DE CAPSULE CARDIAQUE AUTONOME LEADLESS ET UN PROCÉDÉ D'ASSEMBLAGE CORRESPONDANT**
AUF PIEZOELEKTRISCHEM BLATT MONTIERTE TRÄGHEITSGEWICHTSPENDELANORDNUNG, INSBESONDERE FÜR EINEN DRAHTLOSEN ENERGIEREKUPERATOR EINER AUTONOMEN HERZKAPSEL UND EIN ENTSPRECHENDES VERFAHREN ZUM ZUSAMMENBAU
PENDULUM ASSEMBLY WITH INERTIAL MASS MOUNTED ON A PIEZOELECTRIC BLADE, IN PARTICULAR FOR A LEADLESS AUTONOMOUS CARDIAC CAPSULE ENERGY HARVESTER AND A CORRESPONDING ASSEMBLY METHOD

(43) Date de publication de la demande: 03.04.2024
(73) Titulaire: CAIRDAC, 92160 Antony (FR)
(72) Inventeur: REGNIER, Willy, 91160 Longjumeau (FR); DOHIN, Julien, 92170 Vanves (FR); NGUYEN-DINH, An, 37520 La Riche (FR)
(74) Mandataire: Dupuis-Latour, Dominique

(56) Documents cités:
- CN-A- 104 393 787
- CN-A- 107 355 332
- CN-A- 108 649 837
- CN-A- 112 187 102
- US-A1- 2009 174 289

## Description

### CONTEXTE DE L'INVENTION

### Domaine de l'invention

L'invention concerne les récupérateurs d'énergie, également dénommés "harvesters" ou "scavengers", qui recueillent l'énergie mécanique résultant de mouvements divers qu'ils subissent et convertissent cette énergie mécanique en énergie électrique.

Elle concerne plus spécifiquement les récupérateurs de type dit "PEH" (*Piezoelectric Energy Harvester*), qui utilisent comme transducteur mécano-électrique une lame piézoélectrique oscillante couplée à une masse mobile inertielle.

L'invention sera décrite plus particulièrement dans une application de ces récupérateurs d'énergie à des dispositifs médicaux autonomes, notamment les dispositifs de type capsule implantable autonome, en particulier ceux de ces dispositifs qui sont destinés à être implantés dans une cavité cardiaque.

Cette application, si elle est particulièrement avantageuse, ne doit toutefois pas être considérée comme limitative de l'invention, dont les enseignements peuvent être appliqués à de nombreux autres types de dispositifs autonomes incorporant un récupérateur d'énergie de type PEH, que ces dispositifs soient implantables ou non, qu'il s'agisse de dispositifs médicaux ou non.

### Description de la technique antérieure

Dans le domaine des implants médicaux, les récents progrès en matière de miniaturisation de dispositifs actifs et les progrès des sciences du vivant permettent dorénavant le développement d'une grande variété de systèmes miniaturisés implantables totalement autonomes, à des fins de surveillance, de diagnostic ou de traitement. Ces dispositifs mettent en oeuvre des procédures d'implantation moins invasives, plus de confort, des performances accrues et souvent ouvrent l'accès à des nouveaux types de diagnostics et traitements.

Lorsqu'elle est appliquée au domaine des implants médicaux, l'invention concerne plus précisément ceux de ces implants qui incorporent un système d'autoalimentation comprenant un récupérateur d'énergie mécanique associé à un organe de stockage d'énergie intégré tel qu'une batterie rechargeable ou une capacité à hautes performances.

En effet, l'un des aspects critiques de ces dispositifs miniaturisés est celui de l'autonomie électrique. La durée de vie d'un tel implant étant d'environ 8 à 10 ans, compte tenu des très faibles dimensions il n'est pas possible d'utiliser une batterie conventionnelle, même à forte densité.

Le récupérateur d'énergie pallie cet inconvénient en recueillant l'énergie mécanique résultant des mouvements divers subis par le corps du dispositif implanté. Ces mouvements peuvent avoir pour origine un certain nombre de phénomènes se produisant par exemple au rythme des battements cardiaques tels que les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythme des variations de débit.

L'énergie mécanique recueillie par le récupérateur est convertie en énergie électrique (tension ou courant) au moyen d'un transducteur mécanoélectrique approprié, pour assurer l'alimentation des divers circuits et capteurs du dispositif et la recharge de l'organe de stockage d'énergie. Ce système d'alimentation permet au dispositif de fonctionner en parfaite autonomie électrique pendant toute sa durée de vie.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules *leadless*", pour les distinguer des électrodes ou capteurs disposés à l'extrémité distale d'une sonde (*lead*) parcourue sur toute sa longueur par un ou plusieurs conducteurs reliés à un générateur connecté à l'extrémité opposée, proximale.

L'invention n'est toutefois pas limitée à un type particulier de capsule, ni même d'implant leadless, et elle est applicable indifféremment à de nombreux autres types de dispositifs autonomes, quelle que soit leur destination fonctionnelle, cardiaque ou autre, médicale ou non.

Dans le cas d'une application cardiaque, la capsule leadless surveille en continu le rythme du patient et délivre si nécessaire au coeur des impulsions électriques de stimulation, de resynchronisation et/ou de défibrillation en cas de troubles du rythme détecté par la capsule. La capsule comprend par ailleurs divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Le WO 2019/001829 A1 (Cairdac) décrit un exemple d'une telle capsule leadless intracardiaque.

L'invention concerne plus précisément les capsules ou dispositifs implantables analogues dont le récupérateur d'énergie est du type PEH, c'est-à-dire utilisant un transducteur piézoélectrique ou "PZT" (*PieZoelectric Transducer*) et un ensemble pendulaire inertiel soumis aux sollicitations externes décrites plus haut. L'ensemble pendulaire inertiel comprend une masse mobile logée dans le corps de la capsule, dite "masse sismique" ou "masse inertielle", qui est entrainée au gré des mouvements de la capsule soumise en permanence aux diverses sollicitations externes décrites plus haut. Après chacune de ces sollicitations, la masse inertielle, qui est couplée à un élément élastiquement déformable, oscille à une fréquence propre d'oscillation libre.

L'énergie mécanique de l'oscillation est convertie en énergie électrique par un transducteur mécanoélectrique produisant un signal électrique. Ce transducteur mécanoélectrique peut en particulier être un PZT sollicité de façon cyclique en flexion de manière à engendrer au sein du matériau qui le constitue des charges électriques qui sont récupérées en surface du composant pour être utilisées par le système d'autoalimentation de la capsule leadless. Le PZT se présente le plus souvent sous la forme d'une lame encastrée à l'une de ses extrémités et couplée à la masse inertielle à son autre extrémité, qui est libre.

Le signal électrique en sortie du transducteur est délivré à un circuit de gestion de l'alimentation de la capsule, qui redresse et régule le signal électrique pour délivrer en sortie une tension ou un courant continus stabilisés permettant d'assurer l'alimentation des divers circuits électroniques et capteurs de la capsule, ainsi que la recharge de l'organe de stockage d'énergie.

La structure mécanique d'un tel récupérateur d'énergie de type PEH est notamment décrite en détail dans le WO 2018/122244 A1 (Sorin CRM/Regnier).

On notera que le terme de "lame" doit être entendu dans son sens le plus large, à savoir une bande mince et plate de forme allongée, étant entendu que la forme de cette bande n'est pas nécessairement rectangulaire ni son épaisseur constante (comme dans la description du mode de réalisation particulier qui sera donnée plus bas). Le terme de "lame" au sens de la présente invention recouvre ainsi des éléments pouvant présenter une largeur et/ou une épaisseur qui ne sont pas constantes en direction longitudinale, ainsi qu'éventuellement une déformabilité pouvant aller au-delà d'un unique degré de liberté en flexion.

Dans les structures de PEH proposées jusqu'à présent, par exemple par le WO 2018/122244 A1 précité, la masse inertielle est constituée de deux demi-masses identiques, agencées symétriquement de part et d'autre de la lame PZT. Ces deux demi-masses forment ensemble un tronc de cône et sont fixées à l'extrémité libre de la lame de chaque côté de celle-ci par collage. Un autre exemple se trouve dans le brevet CN104393787. Le document divulgue une lame de transducteur avec deux demi masses. Les demi masses ont une orifice, et sont connectées par une pièce transversale de pontage. Cependant, le document ne divulgue pas le matériau piézoélectrique, et la pièce transversale ne passe pas à travers du matériau piézoélectrique.

La conicité de la surface extérieure de la masse inertielle permet d'optimiser l'espace disponible avant d'entrer en contact avec l'intérieur du tube contenant le PEH. Cette géométrie n'est toutefois pas exhaustive et peut être adaptée à son environnement pour optimiser le rapport masse/encombrement.

Le matériau utilisé pour la masse inertielle est un métal, généralement un tungstène moulé, qui présente une masse volumique élevée pour un coût de revient maîtrisé, et les dimensions de la masse sismique sont ajustées en fonction du poids final nécessaire au mode vibratoire souhaité, compte tenu de la géométrie et de l'élasticité de la lame PZT.

Le problème de l'invention trouve sa source dans les difficultés rencontrées du fait du mode d'assemblage de la masse inertielle à la lame PZT, exécuté par collage du métal de chacune des demi-masses sur les faces de la lame PZT en céramique.

En premier lieu, la présence d'une matière chimique de liaison à l'interface métal/céramique a une incidence sur la durée de vie du PEH. Même avec une maîtrise parfaite des colles et de leur mise en oeuvre, il n'a pas jusqu'à présent été possible de garantir une durée de vie supérieure à dix ans sans risque de défaillance.

Ce chiffre de dix ans (correspondant à environ 30 millions de cycles cardiaques) est celui habituellement retenu pour les stimulateurs cardiaques conventionnels, dont il est de toute façon nécessaire de remplacer le générateur à cette échéance compte tenu de l'épuisement de la pile intégrée d'alimentation ; en revanche, dans le cas d'un stimulateur leadless, difficile à explanter pour le remplacer par un appareil neuf, il serait nécessaire de pouvoir garantir une longévité bien supérieure, typiquement de 20 ans de fonctionnement continu sans défaillance.

Les colles employées jusqu'à présent ne permettent toutefois pas de garantir une telle performance, même pour celles qui ne se dégradent que très peu au cours du temps.

Un deuxième problème réside dans la difficulté qu'il y a à bien maîtriser le processus de collage au moment de la fabrication du PEH. Ce processus est en soi très délicat à mettre en oeuvre du fait des dimensions très réduites des pièces, de la nécessité d'opérer sous atmosphère contrôlée, et d'éviter toute pollution chimique par des contaminants susceptibles de modifier les propriétés de tenue au vieillissement du collage effectué.

Un troisième problème réside, lors de ce processus de collage, dans la difficulté particulière qu'il y a à maîtriser parfaitement la quantité de colle utilisée : une quantité insuffisante de colle diminue bien évidemment la solidité du collage final obtenu, mais à l'inverse un excès de colle se traduit par un épanchement de la colle au-delà de l'interface métal/céramique, avec un risque d'altérer la flexibilité de la lame PZT (qui perd sa flexibilité à l'endroit où.la colle a débordé) avec augmentation de la fréquence de vibration propre de l'ensemble pendulaire, et par voie de conséquence un dérèglement du système induisant une moindre récupération d'énergie par le PEH, toutes choses égales par ailleurs.

Le but de l'invention est de proposer une nouvelle structure de module PEH, et un nouveau procédé d'assemblage d'une telle structure, qui pallient les difficultés et limitations que l'on vient d'exposer, en permettant notamment :
de garantir une longévité du PEH pouvant atteindre 20 ans ;
d'offrir une technique de montage simplifiée, économique et non opérateur-dépendante ; et
d'obtention un PEH avec des caractéristiques vibratoires parfaitement maîtrisées et, de fait, un rendement optimisé de la fonction de récupération d'énergie.

### RÉSUMÉ DE L'INVENTION

L'invention est défini par le composant selon la revendication 1, et le procédé selon la revendication 5. Pour résoudre ces problèmes et atteindre les buts exposés ci-dessus, l'invention propose un ensemble pendulaire destiné à un module PEH, cet ensemble pendulaire comprenant, de façon en elle-même connue notamment d'après le WO 2018/122244 A1 précité, une lame de transducteur piézoélectrique, PZT, la lame PZT s'étendant en direction axiale entre une extrémité proximale, encastrée dans une pièce d'encastrement, et une extrémité distale libre et étant élastiquement déformable en flexion, et une masse inertielle, montée à l'extrémité distale libre de la lame PZT et mobile en direction transversale, la masse inertielle comprenant deux demi-masses agencées de part et d'autre de la lame PZT à l'extrémité distale libre de celle-ci. L'ensemble pendulaire est apte à convertir une énergie mécanique produite par des oscillations de l'ensemble pendulaire sous l'effet de sollicitations externes subies par le module en un signal électrique oscillant recueilli par des électrodes de surface de la lame PZT.

De façon caractéristique de l'invention, l'ensemble pendulaire comprend une liaison mécanique des deux demi-masses l'une à l'autre de part et d'autre de la lame PZT, la liaison mécanique étant apte à pincer la lame PZT entre les deux demi-masses pour assujettir la masse inertielle à la lame PZT, l'assemblage étant dépourvu de colle entre les demi-masses et la lame PZT.

Selon l'invention:
- la liaison mécanique des deux demi-masses l'une à l'autre de part et d'autre de la lame PZT comprend au moins une pièce transversale de pontage rapportée ;
- la pièce transversale de pontage est agencée à l'extrémité distale et/ou proximale des deux demi-masses ;
- l'ensemble pendulaire comprend en outre au moins un point de soudure reliant la pièce transversale de pontage à chacune des deux demi-masses ;
- la lame PZT comprend un orifice et/ou une encoche aptes à être traversés par la pièce transversale de pontage ;
- la liaison mécanique des deux demi-masses l'une à l'autre de part et d'autre de la lame PZT comprend une pièce de cerclage entourant totalement ou partiellement et enserrant les deux demi-masses à leur extrémité distale et/ou proximale ;
- les deux demi-masses sont des pièces emboitables ayant des formes respectives conjuguées complémentaires ; et/ou
- l'ensemble pendulaire comprend en outre au moins un point de soudure entre les pièces emboitables.

L'invention a également pour objet un procédé d'assemblage d'un tel ensemble pendulaire, comprenant les étapes suivantes :
a) obtention d'une lame PZT ;
b) mise en place à l'extrémité distale libre de la lame PZT de deux demi-masses formant la masse inertielle, de part et d'autre de la lame PZT ; et
c) création d'une liaison mécanique des deux demi-masses l'une à l'autre de part et d'autre de la lame PZT, sans apport de colle entre la lame PZT et les demi-masses, la liaison mécanique étant apte à pincer la lame PZT entre les deux demi-masses pour assujettir la masse inertielle à la lame PZT.

Selon l'invention de ce procédé :
- l'étape c) comprend : c1) la mise en place d'au moins une pièce transversale de pontage à l'extrémité distale et/ou proximale des deux demi-masses ; et c2) le soudage de l'au moins une pièce transversale de pontage à chacune des deux demi-masses ;
- les deux demi-masses sont des pièces emboitables ayant des formes respectives conjuguées complémentaires, et l'étape c) comprend le soudage l'une à l'autre des deux pièces emboitables, ou bien comprend : c1) la déformation en température de l'une des deux pièces emboitables ; c2) l'assemblage des deux pièces emboitables l'une avec l'autre ; et c3) le retour à température ambiante, pour assujettir entre elles par jeu négatif les deux pièces emboitables.

L'invention a également pour objet un PEH comprenant un tube enveloppe allongé et, logé à l'intérieur du tube, un ensemble pendulaire tel que ci-dessus.

L'invention porte également sur un dispositif autonome incorporant dans un corps de dispositif un module PEH tel que ci-dessus et comprenant :
- un ensemble électronique ;
- un module PEH tel que ci-dessus, produisant en sortie un signal électrique ;
- un circuit de gestion d'alimentation, apte à redresser et réguler le signal électrique produit par le module PEH, pour délivrer en sortie une tension ou un courant continus d'alimentation stabilisés ; et
- un organe de stockage d'énergie pour l'alimentation de l'ensemble électronique,

Ladite tension ou ledit courant continus stabilisés délivrés par le circuit de gestion d'alimentation servent à l'alimentation de l'ensemble électronique et/ou à la recharge de l'organe de stockage d'énergie du dispositif autonome.

Ce dispositif autonome peut notamment être un dispositif médical actif de type capsule leadless, comprenant un corps de capsule avec un élément d'ancrage à une paroi d'un organe d'un patient, et dans laquelle les sollicitations externes auxquelles est soumis l'ensemble pendulaire du module PEH sont des sollicitations appliquées au corps de capsule sous l'effet de mouvements de ladite paroi et/ou de variations de débit d'un flux dans le milieu environnant.

### DESCRIPTION SOMMAIRE DES DESSINS

On va maintenant décrire un exemple de réalisation de la présente invention en référence aux dessins annexés, où les mêmes références désignent d'une figure à l'autre des éléments identiques ou fonctionnellement semblables.
La Figure 1 illustre des dispositifs médicaux de type capsule leadless dans leur environnement, avec divers exemples de sites d'implantation dans, sur ou à proximité du coeur d'un patient.
La Figure 2 illustre une capsule leadless implantée au fond du ventricule droit d'un patient.
La Figure 3 montre isolément un ensemble pendulaire de type connu, avec un PZT en forme de lame allongée encastrée à une extrémité et supportant une masse inertielle à son extrémité opposée.
La Figure 4 présente sous forme schématique les principaux blocs fonctionnels constitutifs d'une capsule leadless.
La Figure 5 est une vue en coupe transversale, par un plan axial, du module PEH selon l'invention.
La Figure 6 est une vue éclatée en perspective montrant les différents éléments constitutifs du module PEH de la Figure 5.
La Figure 7 est une vue en coupe, par un plan axial, d'une masse inertielle d'un ensemble pendulaire réalisé selon un premier mode de réalisation de l'invention.
Les Figures 8 et 9 sont des vues perspectives, respectivement éclatée et assemblée, de la masse inertielle de l'ensemble pendulaire illustré Figure 7.
Les Figures 10 et 11 sont des vues perspectives, respectivement éclatée et assemblée, d'une masse inertielle d'un ensemble pendulaire selon un deuxième mode de réalisation de l'invention.
Les Figures 12 et 13 sont des vues perspectives, respectivement éclatée et assemblée, d'une masse inertielle d'un ensemble pendulaire selon un troisième mode de réalisation de l'invention.
Les Figures 14 et 15 sont des vues perspectives, respectivement éclatée et assemblée, d'une masse inertielle d'un ensemble pendulaire selon un quatrième mode de réalisation de l'invention.
La Figure 16 est une vue en coupe, par un plan radial, de la masse inertielle de l'ensemble pendulaire illustré Figures 14 et 15.
Les Figures 17 et 18 illustrent deux étapes d'assemblage d'une capsule leadless avec un module PEH comprenant un ensemble pendulaire selon l'invention.
La Figure 19 illustre la capsule leadless implantable finale obtenue en fin de processus.
La Figure 20 est un organigramme explicitant les différentes étapes du processus d'assemblage d'une capsule leadless implantable comprenant un module PEH avec un ensemble pendulaire selon l'invention.

### DESCRIPTION DÉTAILLÉE DE MODES DE RÉALISATION PRÉFÉRENTIELS DE L'INVENTION

On va maintenant décrire un exemple de réalisation du dispositif de l'invention, dans une application à une capsule implantable autonome destinée à être implantée dans une cavité cardiaque.

Comme on l'a indiqué plus haut, cette application particulière n'est donnée qu'à titre d'exemple de réalisation et n'est pas limitative de l'invention, dont les enseignements peuvent être appliqués à de nombreux autre types de dispositifs autonomes incorporant un récupérateur d'énergie de type PEH, que ces dispositifs soient implantables ou non, qu'il s'agisse de dispositifs médicaux ou non.

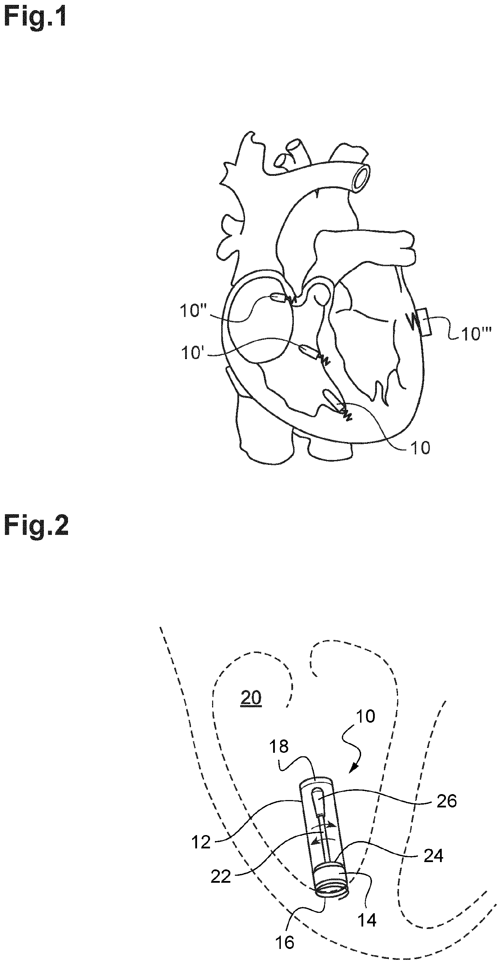

Sur la Figure 1, on a représenté diverses possibilités de sites d'implantation d'un dispositif de type leadless, dans une application à la stimulation cardiaque. Ainsi, la capsule 10 est implantée à l'intérieur d'une cavité du myocarde (implant endocavitaire), par exemple à l'apex du ventricule droit. En variante, la capsule peut être également implantée sur le septum interventriculaire droit, comme en 10', ou encore sur une paroi auriculaire, comme illustré en 10". Le dispositif peut également être une capsule épicardique placée sur une région externe du myocarde, comme illustré en 10‴.

Dans chaque cas, la capsule leadless est fixée à la paroi cardiaque au moyen d'un système d'ancrage saillant pénétrant dans le tissu cardiaque pour le maintien sur le site d'implantation. D'autres systèmes d'ancrage sont utilisables, et ne modifient en aucune façon la mise en oeuvre de la présente invention.

La capsule 10 se présente sous forme extérieure d'un implant avec un corps tubulaire allongé 12 enfermant les divers circuits électroniques et d'alimentation de la capsule ainsi qu'un récupérateur d'énergie à ensemble pendulaire. Les dimensions typiques des capsules connues sont un diamètre de l'ordre de 6 mm pour une longueur d'environ 25 à 40 mm.

Le corps tubulaire 12 comporte à son extrémité avant (distale) 14 un élément d'ancrage saillant, par exemple une vis hélicoïdale 16 pour maintenir la capsule sur le site d'implantation. D'autres systèmes d'ancrage sont utilisables, et ne modifient en aucune façon la mise en oeuvre de la présente invention. L'extrémité opposée (proximale) 18 de la capsule 10 est une extrémité libre, qui est seulement pourvue de moyens (non représentés) de liaison temporaire à un cathéter-guide ou autre accessoire d'implantation utilisé pour la mise en place ou l'explantation de la capsule, qui est ensuite désolidarisé de cette dernière.

Dans l'exemple illustré Figure 2, la capsule leadless 10 est un implant endocavitaire implanté à l'intérieur d'une cavité 20 du myocarde, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une région externe du myocarde, ces différents modes d'implantation ne modifiant en aucune façon la mise en oeuvré de la présente invention. Pour assurer les fonctions de détection/stimulation, une électrode (non représentée) en contact avec le tissu cardiaque au site d'implantation assure le recueil des potentiels de dépolarisation cardiaque et/ou l'application d'impulsions de stimulation. Dans certaines formes de réalisation, la fonction de cette électrode peut être assurée par la vis d'ancrage 16, qui est alors une vis active, électriquement conductrice et reliée au circuit de détection/stimulation de la capsule.

La capsule leadless 10 est par ailleurs dotée d'un module récupérateur d'énergie dit "PEH", comprenant un ensemble pendulaire inertiel qui oscille à l'intérieur de la capsule au gré des diverses sollicitations externes auxquelles la capsule est soumise. Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises par les tissus cardiaques.

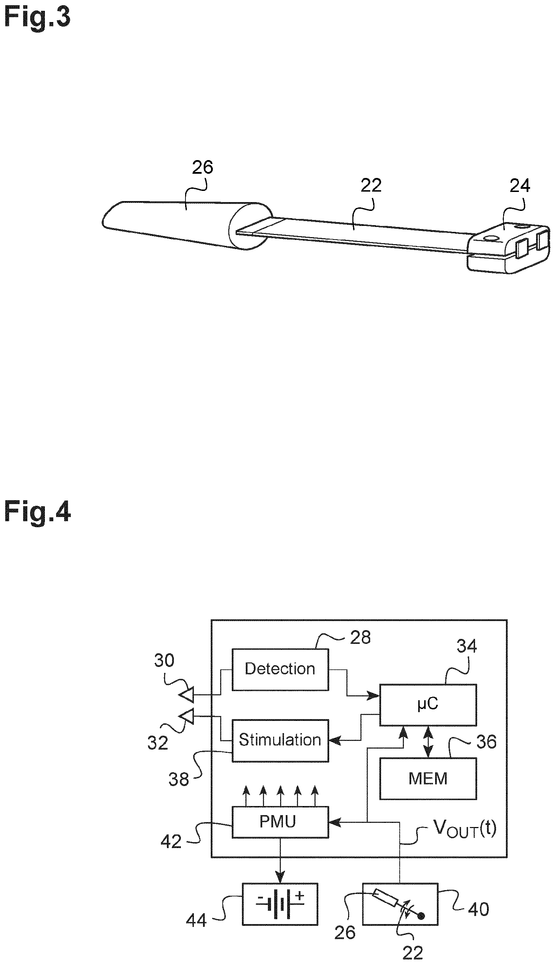

L'ensemble pendulaire, illustré isolément Figure 3, est constitué d'une lame piézoélectrique 22 assujettie à une pièce d'encastrement 24 à l'une de ses extrémités (ci-après "extrémité proximale" de la lame) et dont l'extrémité opposée, libre (ci-après "extrémité distale" de la lame) est couplée à une masse inertielle mobile 26. La lame piézoélectrique 22 est une lame flexible élastiquement déformable qui constitue avec la masse inertielle 26 un système pendulaire de type masse-ressort. Du fait de son inertie, la masse 26 soumet la lame 22 à une déformation de type vibratoire de part et d'autre d'une position neutre ou non déformée correspondant à une position stable de repos en l'absence de toute sollicitation. Les dimensions typiques minimales des lames PZT des dispositifs connus de ce type sont de l'ordre de 25 mm de long pour une largeur de 5 mm environ.

De fait, pour ce qui est de son comportement mécanique, cet ensemble peut être assimilé à une structure de type "poutre encastrée-libre", présentant une fréquence d'oscillation propre qui est ici la fréquence sur laquelle oscille le système masse-ressort. On notera que cette fréquence d'oscillation propre, typiquement de l'ordre de quelques dizaines de hertz, est notablement supérieure à la fréquence des sollicitations cycliques externes qui correspondent à la fréquence des battements cardiaques (quelques hertz tout au plus). Ainsi, à chaque contraction cardiaque la masse inertielle (ou autre organe mécanique fonctionnellement analogue) sera sollicitée avec une amplitude plus ou moins importante, puis le système pendulaire oscillera plusieurs fois avec des amplitudes décroissantes (rebonds caractéristiques d'une oscillation périodique amortie), et enfin se stabilisera jusqu'au battement cardiaque suivant, où le cycle sollicitation/oscillations se reproduira de façon comparable.

La lame 22 assure en outre, par effet piézoélectrique, une fonction de transducteur mécanoélectrique permettant de convertir en charges électriques la contrainte mécanique de flexion qui lui est appliquée. Ces charges sont collectées par des électrodes à la surface de la lame de manière à produire un signal électrique qui, après redressement, stabilisation et filtrage, alimentera les divers circuits électroniques de la capsule.

La Figure 4 est un synoptique des divers circuits électriques et électroniques intégrés à la capsule leadless, présenté sous forme de blocs fonctionnels.

Le bloc 28 désigne un circuit de détection de l'onde de dépolarisation cardiaque, qui est relié à une électrode de cathode 30 en contact avec le tissu cardiaque et à une électrode d'anode 32 associée, par exemple une électrode annulaire formée sur le corps tubulaire de la capsule. Le bloc de détection 28 comprend des filtres et des moyens de traitement analogique et/ou numérique du signal recueilli. Le signal ainsi traité est appliqué à l'entrée d'un microcalculateur 34 associé à une mémoire 36. L'ensemble électronique comporte également un circuit de stimulation 38 opérant sous le contrôle du microcalculateur 34 pour délivrer au système d'électrodes 30, 32 des impulsions de stimulation du myocarde.

Il est par ailleurs prévu un circuit récupérateur d'énergie ou PEH 40, constitué par l'ensemble pendulaire formé par la lame piézoélectrique 22 et la masse inertielle 26 décrits plus haut en référence aux Figures 2 et 3. Comme la lame piézoélectrique 22 assure aussi une fonction de transducteur mécano-électrique, elle convertit en charges électriques les sollicitations mécaniques subies et produit un signal électrique variable V_{OUT}(t), qui est un signal alternatif oscillant à la fréquence d'oscillation libre de l'ensemble pendulaire lame 22/masse 26, et au rythme des battements successifs du myocarde auquel la capsule est couplée.

Le signal électrique variable V_{OUT}(t) est délivré à un circuit de gestion de l'énergie ou PMU 42. Le PMU 42 redresse et régule le signal V_{OUT}(t) de manière à produire en sortie une tension ou un courant continus stabilisés servant à l'alimentation des divers circuits électroniques et à la recharge de la batterie intégrée 44.

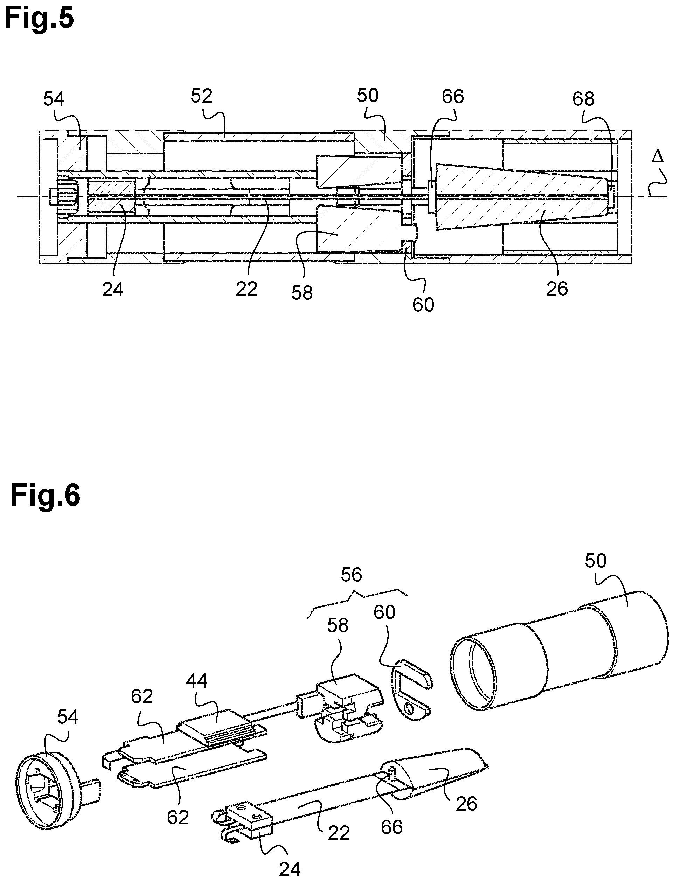

D'autre part, la lame est avantageusement une lame de type bimorphe, c'est-à-dire capable de générer de l'énergie sur ses deux faces lorsqu'elle est soumise à une déformation. Ces propriétés de transduction sont typiques d'un matériau piézoélectrique tel que les céramiques PZT ou les monocristaux de type PMN-PT, titanate de baryum ou niobate de lithium. Sur les Figures 5 et 6 on a représenté les principaux éléments constitutifs d'un module PEH selon l'invention.

Ces différents éléments sont logés à l'intérieur d'un tube enveloppe 50, qui est généralement un tube métallique (pour permettre des opérations de soudage qui seront décrites plus bas), de préférence en titane en raison de l'excellente biocompatibilité de ce métal.

Un tube enveloppe particulièrement adapté à la réalisation d'une capsule leadless est décrit notamment dans le EP 3 730 185 A1 (Cairdac), correspondant au US 2020/338241 A1 (Regnier et al.), qui illustre notamment un tube composite métal/céramique comprenant une partie centrale (52 sur la Figure 5) en matériau céramique transparent aux radiofréquences, de manière à permettre une communication sans fil entre des circuits électroniques situés à l'intérieur du tube et l'environnement extérieur, le reste du tube étant en matériau métallique tel que le titane et le tout formant un ensemble monobloc tubulaire.

Le tube enveloppe 50 loge l'ensemble pendulaire constitué de la lame 22 maintenue côté proximal par la pièce d'encastrement 24 et portant côté distal la masse inertielle 26. L'ensemble pendulaire est agencé au centre du tube enveloppe 50 et aligné sur l'axe Δ du tube.

Dans la suite, on entendra par "direction axiale" la direction de plus grande longueur de la lame et par "direction transversale" la direction de déformation de la lame, direction qui est située dans un plan radial et qui est perpendiculaire à la direction axiale Δ ; la direction perpendiculaire aux directions axiale et transversale sera dénommée "direction latérale".

La pièce d'encastrement 24 est maintenue dans le tube par une monture 54 assujettie au tube, notamment une monture en un matériau métallique tel que le titane, susceptible d'être soudé au tube en périphérie de manière à assujettir au tube 50 la monture 54, et donc l'encastrement 24 et la lame 22.

Le EP 3 892 325 A1 (Cairdac), correspondant au US 2021/316148 A1 (Regnier et al.), décrit en détail un exemple de pièce d'encastrement et de monture, et on pourra se référer à ce document pour de plus amples détails.

Le tube 50 loge également une ou plusieurs cartes de circuit imprimé (PCB) 62, dans l'exemple illustré deux PCB 62 dont l'un porte la batterie 44. Ces deux PCB sont reliés l'un à l'autre par une nappe de conducteurs flexibles et supportés à chacune de leurs extrémités respectivement côté distal par un insert 56 et côté proximal par la monture 54.

La configuration de ces PCB de part et d'autre de la lame 22, et la manière dont ils sont réunis par une nappe flexible et supportés entre un élément proximal et un élément distal sont décrits notamment dans le US 2019/381325 A1 précité, auquel on pourra se référer pour de plus amples détails.

L'insert 56 est par exemple un insert de symétrisation tel que celui décrit dans la demande EP 22315101.0 du 10.05.2022 au nom du demandeur pour un "*Récupérateur d'énergie piézoélectrique avec lame à débattement contrôlé, notamment pour l'alimentation d'une capsule cardiaque autonome leadless*". Cet insert de symétrisation permet de préserver l'amplitude maximale d'oscillation de la lame en évitant qu'elle ne soit réduite par un positionnement sous-optimal de l'ensemble pendulaire dans le corps du module, notamment du fait d'un positionnement imparfait (décentrage, désalignement) de la masse inertielle 26.

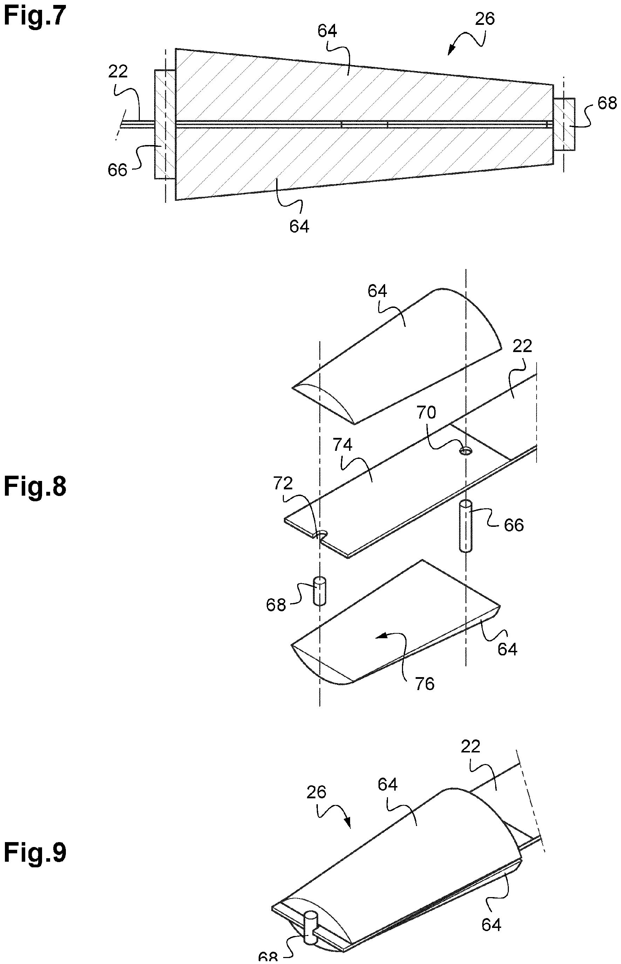

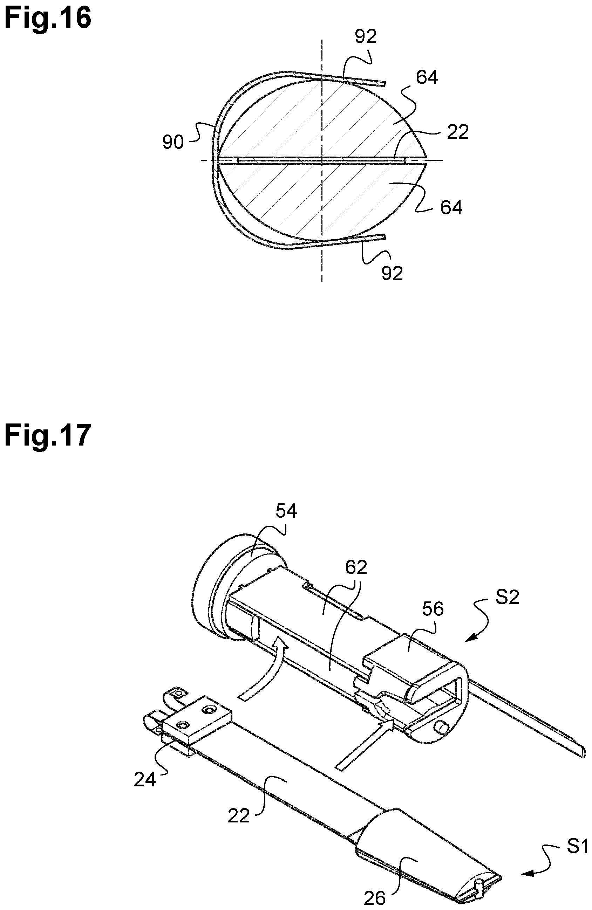

L'invention concerne plus précisément la manière dont est réalisée et assemblée la masse inertielle 26 de l'ensemble pendulaire de la capsule leadless que l'on vient de décrire. Les Figures 7 à 16 illustrent divers modes de réalisation selon l'invention, et les Figures 17 à 20 illustrent le procédé de réalisation d'une capsule leadless munie d'un tel ensemble pendulaire.

La masse inertielle 26 comprend, de manière en elle-même connue, deux demi-masses 64, 64, généralement de forme tronconique, agencées de part et d'autre de la lame PZT 22 à l'extrémité distale libre de celle-ci.

De façon caractéristique de l'invention, il est prévu une liaison mécanique des deux demi-masses 64, 64 l'une à l'autre, de part et d'autre de la lame PZT 22.

Cette liaison mécanique entre les deux demi-masses 64, 64 est conçue pour pincer la lame PZT 22 entre les deux demi-masses 64, 64 de manière que l'ensemble soit solidarisé en excluant toute présence de colle à l'interface demi-masse métallique/lame PZT céramique.

Les Figures 7 à 9 illustrent un premier mode de réalisation de cette liaison mécanique selon l'invention.

Les deux demi-masses 64, 64 sont réunies l'une à l'autre par deux pièces transversales de pontage 66, 68 agencées respectivement à l'extrémité proximale et à l'extrémité distale des demi-masses.

Ces pièces de pontage sont par exemple des axes ou tiges métalliques traversant respectivement un trou ou une encoche 70, 72 de la lame PZT 22, pratiqués dans une région d'extrémité 74 de la lame PZT qui est dépourvue d'électrodes de surface ou, en variante, en ajoutant un élément isolant. Ceci évite que, avec une pièce de pontage 66 ou 68 en matériau électriquement conducteur, cette pièce court-circuite des électrodes de la lame PZT en risquant d'annuler les charges positive et négative générées de part et d'autre de la lame PZT. D'autre part, les demi-masses 64, 64 présentent, de leur côté tourné vers la lame PZT 22 une face plane 76 apte à être appliquée contre la région d'extrémité 74 de cette lame PZT.

Les pièces de pontage 66, 68 sont en un matériau apte à être soudé à chacune des extrémités des demi-masses 64, 64, par exemple en acier inoxydable 316L ou, de préférence, en tungstène lorsque les demi-masses 64 sont également en tungstène.

Dans ce mode de réalisation, la liaison mécanique des deux demi-masses entre elles est réalisée par : centrage des demi-masses 64, 64 contre la lame PZT 22 ; mise en pression (pincement) des demi-masses contre la lame PZT ; positionnement des pièces de pontage 66, 68 aux extrémités respectivement proximale et distale de chacune des demi-masses ; enfin soudage des pièces de pontage aux demi-masses.

Une fois ce soudage effectué, la lame PZT 22 reste serrée et pincée entre les deux demi-masses 64, 64 et assujettie définitivement à celles-ci, la configuration finale obtenue étant celle illustrée Figure 9.

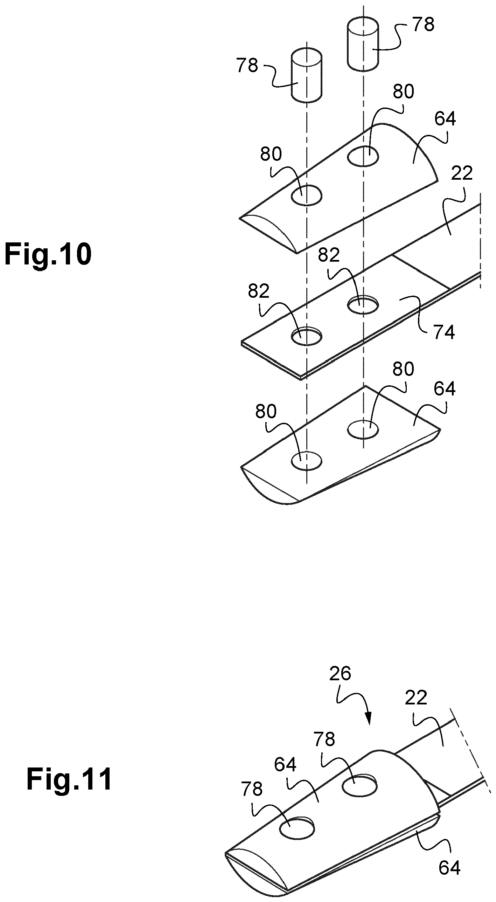

Les Figures 10 et 11 illustrent un second mode de réalisation, qui est une variante du précédent.

Dans ce second mode de réalisation, les pièces de pontage sont des axes 78 transversaux qui sont insérés dans des alésages correspondants 80 des demi-masses 64, 64 et qui traversent des orifices 82, 82 pratiqués dans une zone centrale de la lame PZT 22 (au lieu d'une zone périphérique comme dans le premier mode de réalisation), dans la région 74 dépourvue d'électrodes.

Les axes 78 sont de préférence en un matériau métallique, très préférentiellement en tungstène tout comme les demi-masses 64, pour bénéficier de la masse volumique très élevée de ce métal. En variante, les axes centraux 78 peuvent également être en un matériau de synthèse, par exemple en PEEK (polyétheréthercétone) ou autre matériau synthétique.

Lorsque les axes 78 sont en métal, la liaison mécanique des axes 78 aux demi-masses 64 est avantageusement une liaison soudée, comme dans le premier mode de réalisation ; en variante, cette liaison peut être également une liaison collée, notamment dans le cas de pièces de pontage 78 en matériau synthétique (on notera que, dans ce mode de réalisation, le collage se fait entre les pièces de pontage 78 et les demi-masses 64, mais qu'il n'y a aucun collage de ces demi-masses à la lame PZT 22).

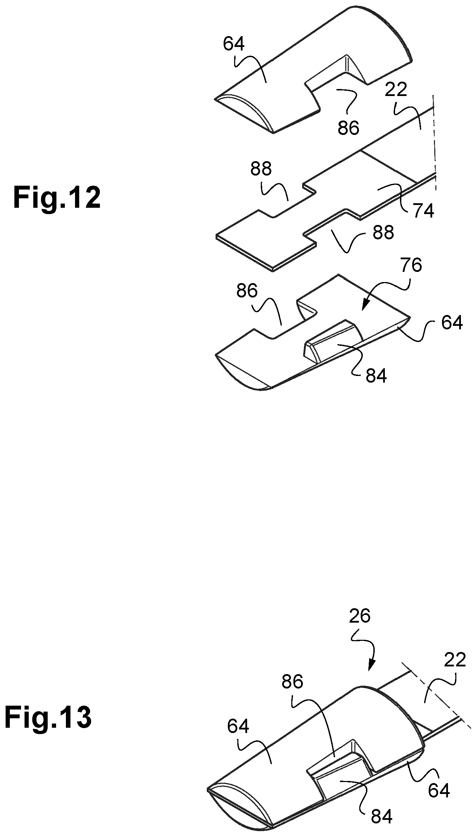

Les Figures 12 et 13 illustrent un troisième mode de réalisation de l'invention qui ne nécessite pas l'utilisation d'élément rapporté pour réaliser la liaison mécanique des deux demi-masses entre elles, à la différence des pièces de pontage des deux modes de réalisation précédents.

Dans ce troisième mode de réalisation, les demi-masses sont réalisées sous forme de deux pièces identiques emboitables, montées en opposition de part et d'autre de la lame PZT 22. Plus précisément, chacune des demi-masses 64 comporte, du côté de la face plane 76 destinée à venir en contact avec la lame PZT 22, un bossage 84 apte à venir s'emboîter dans une échancrure de forme conjuguée 86 de la demi-masse opposée située de l'autre côté de la lame PZT 22. Cette demi-masse opposée comprend également un bossage apte à venir s'emboîter dans une échancrure 86 de la première demi-masse. La lame PZT 22 comporte deux encoches 88 à l'emplacement des bossages 84.

On notera à cet égard que les demi-masses emboitables 64 ne sont pas nécessairement identiques ; bien au contraire, pour faciliter l'assemblage il peut être avantageux de prévoir pour les bossages 84 et les échancrures conjuguées 86 des positionnements et dimensions différents entre les deux demi-masses 64.

Ce mode de réalisation présente également l'avantage de permettre, grâce aux formes conjuguées des bossages 84 et des échancrures 86 d'un assemblage et d'un centrage simples des deux demi-masses sur la lame PZT 22.

Une fois les deux demi-masses réunies, l'ensemble présente la configuration illustrée Figure 13 et l'assujettissement définitif des deux demi-masses entre elles peut être obtenu par un ou plusieurs points de soudure, notamment au niveau des interfaces bossage 84/échancrure 86. Dans une variante sans soudage, après avoir déformé en température l'une des deux pièces emboîtables, les deux pièces emboîtables sont réunies l'une à l'autre puis on laisse l'ensemble retourner à température ambiante pour assujettir entre elles, par jeu négatif, les deux demi-masses emboîtées.

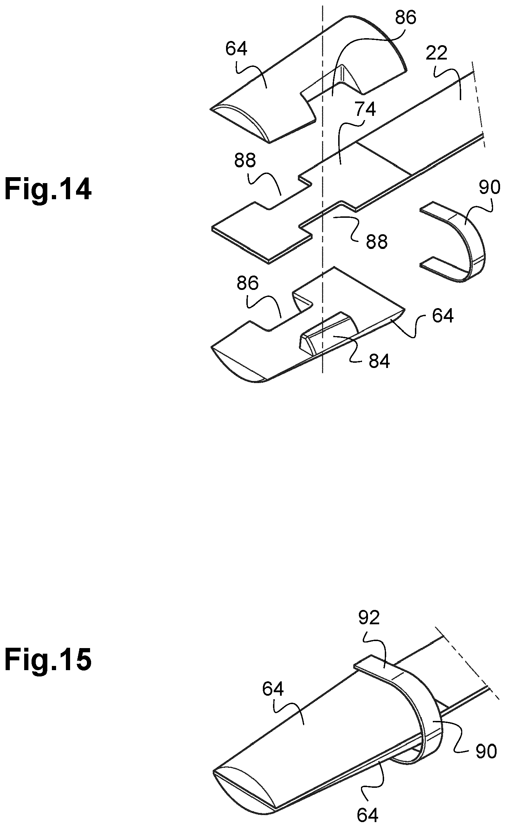

Les Figures 14 à 16 illustrent un quatrième mode de réalisation de l'invention, qui est une variante du précédent.

Dans ce mode de réalisation, pour éviter d'avoir à souder ou à déformer en températures les deux demi-masses, on insère une pièce de cerclage ou frette 90 qui vient entourer tout ou partie des deux demi-masses après que celles-ci ont été assemblées de part et d'autre de la lame PZT 22. La pièce de cerclage 90 comprend des faces d'appui 92 qui viennent pincer l'une contre l'autre les deux demi-masses 64, avec un effort contrôlé, de préférence inférieur à 10 N. La pièce de cerclage 90 peut être réalisée en un matériau métallique (par exemple inox ou nitinol) ou en matière synthétique (par exemple en PEEK).

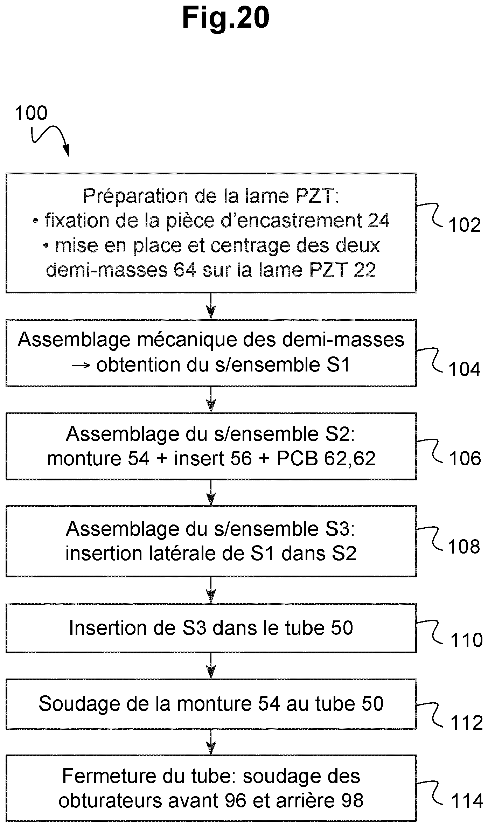

En référence aux Figures 17 à 19 et à l'organigramme de la Figure 20 présentant les différentes étapes du processus, on va maintenant exposer la manière dont sont fabriqués et assemblés le module PEH comprenant l'ensemble pendulaire muni de la masse sismique que l'on vient de décrire, ainsi que la capsule leadless complète intégrant un tel module.

La première étape (bloc 102 de l'organigramme 100 de la Figure 20), consiste à préparer la lame PZT 22 en la fixant à son extrémité proximale sur la pièce d'encastrement 24 et en mettant en place et en centrant, à son extrémité distale, les deux demi-masses 64 de part et d'autre de la lame selon l'un des modes de réalisation que l'on a décrit plus haut.

L'étape suivante (bloc 104) consiste à réaliser la liaison mécanique définitive des deux demi-masses entre elles en prenant en sandwich la lame PZT. On obtient ainsi un premier sous-ensemble S1 (Figure 17) constitué de la lame PZT 22, de la pièce d'encastrement 24 à son extrémité proximale, et de la masse inertielle 26 à son extrémité distale.

L'étape suivante (bloc 106) consiste à assembler un sous-ensemble S2 (Figure 17) regroupant la monture 54, l'insert 56, et les deux PCB 62 montés entre ces deux éléments 54 et 56.

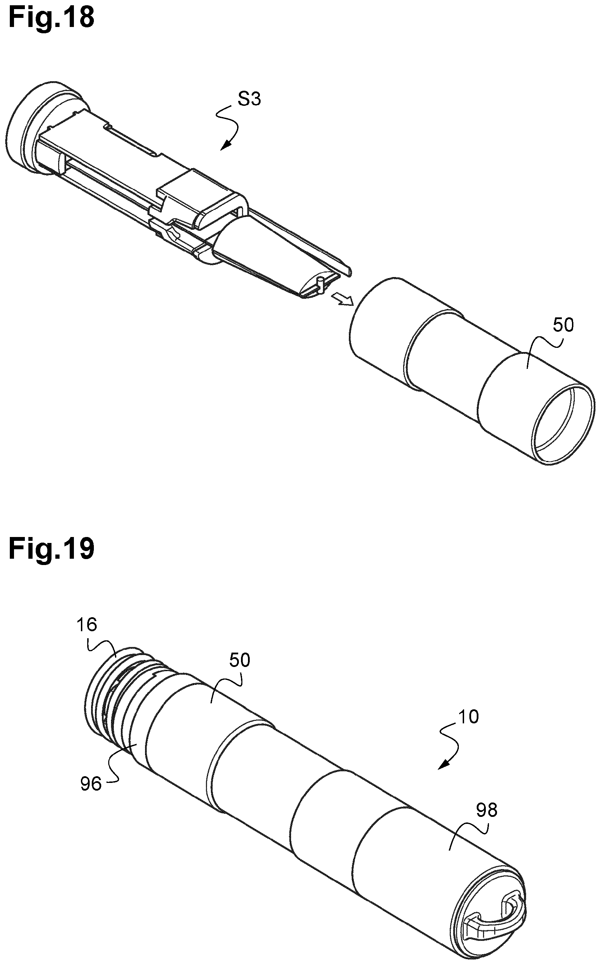

L'étape suivante (bloc 108) consiste à réunir les sous-ensembles S1 et S2 en un sous-ensemble S3 (Figure 18) par introduction du sous-ensemble S1 dans l'espace latéral ménagé entre l'insert 56, la monture 54 et les deux PCB 62 du sous-ensemble S2.

L'étape suivante (bloc 110) consiste à introduire ce sous-ensemble S3 dans le tube enveloppe 50 par translation axiale (Figure 18). La monture 54 est alors soudée au tube 50 (étape 112), par exemple au moyen de tirs laser périphériques.

L'étape finale (bloc 114) consiste à fermer à ses deux extrémités le tube enveloppe 50 contenant l'ensemble pendulaire, par ajout d'un obturateur avant 96 portant la vis d'ancrage 16 de la capsule leadless, et d'un obturateur arrière 98. Ces obturateurs 96 et 98 sont fixés au tube 50 par des soudages laser périphériques. La capsule leadless se présente alors dans son état assemblé final, tel qu'illustré sur la Figure 19.

## Revendications

1. Un ensemble pendulaire destiné à un module de récupération d'énergie, PEH, l'ensemble pendulaire comprenant :
- une lame de transducteur piézoélectrique, PZT, la lame PZT (22) s'étendant en direction axiale entre une extrémité proximale, encastrée dans une pièce d'encastrement (24), et une extrémité distale libre et étant élastiquement déformable en flexion ; et
- une masse inertielle (26), montée à l'extrémité distale libre de la lame PZT (22) et mobile en direction transversale, la masse inertielle (26) comprenant deux demi-masses (64, 64) agencées de part et d'autre de la lame PZT (22) à l'extrémité distale libre de celle-ci,
l'ensemble pendulaire étant apte à convertir une énergie mécanique produite par des oscillations de l'ensemble pendulaire sous l'effet de sollicitations externes subies par le module en un signal électrique oscillant recueilli par des électrodes de surface de la lame PZT (22),
l'ensemble pendulaire comprenant une liaison mécanique des deux demi-masses (64, 64) l'une à l'autre de part et d'autre de la lame PZT (22), la liaison mécanique étant apte à pincer la lame PZT (22) entre les deux demi-masses (64, 64) pour assujettir la masse inertielle (26) à la lame PZT (22), l'assemblage étant dépourvu de colle entre les demi-masses (64, 64) et la lame PZT (22),
**caractérisé en ce que** la liaison mécanique des deux demi-masses (64, 64) l'une à l'autre de part et d'autre de la lame PZT (22) comprend :
au moins une pièce transversale de pontage rapportée (66, 68 ; 78, 78), soudée à chacune des deux demi-masses (64, 64), et/ou
des formes respectives conjuguées complémentaires emboitables (84, 86) des deux demi-masses (64, 64),
**et en ce que** la lame PZT (22) comprend un orifice (70 ; 82, 82) et/ou une encoche (72 ;88, 88) aptes à être traversés :
par l'au moins une pièce transversale de pontage rapportée (66, 68 ; 78, 78) ou, respectivement,
par un bossage (84) des formes conjuguées complémentaires emboitables (84, 86).

2. L'ensemble pendulaire de la revendication 1, dans lequel l'au moins une pièce transversale de pontage rapportée (66, 68) est agencée à l'extrémité distale et/ou proximale des deux demi-masses (64, 64).

3. L'ensemble pendulaire de la revendication 1, dans lequel l'au moins une pièce transversale de pontage rapportée (78, 78) est agencée dans une zone centrale des deux demi-masses (64, 64).

4. L'ensemble pendulaire de la revendication 1, comprenant en outre au moins un point de soudure entre les formes respectives conjuguées complémentaires emboitables (84, 86).

5. Un procédé d'assemblage d'un ensemble pendulaire destiné à un module de récupération d'énergie, PEH, l'ensemble pendulaire comprenant une lame (22) de transducteur piézoélectrique, PZT, élastiquement déformable en flexion et une masse inertielle (26) montée à l'extrémité distale libre de la lame PZT (22) et mobile en direction transversale,
procédé comprenant les étapes suivantes :
a) obtention d'une lame PZT (22) ;
b) mise en place à l'extrémité distale libre de la lame PZT (22) de deux demi-masses (64, 64) formant la masse inertielle (26), de part et d'autre de la lame PZT (22) ; et
c) création d'une liaison mécanique des deux demi-masses (64, 64) l'une à l'autre de part et d'autre de la lame PZT (22), sans apport de colle entre la lame PZT (22) et les demi-masses (64, 64), la liaison mécanique étant apte à pincer la lame PZT (22) entre les deux demi-masses (64, 64) pour assujettir la masse inertielle (26) à la lame PZT (22),
procédé dans lequel l'étape c) comprend :
c1) la mise en place d'au moins une pièce transversale de pontage (66, 68; 78, 78) à l'extrémité distale et/ou proximale des deux demi-masses (64, 64) ; et
c2) le soudage de l'au moins une pièce transversale de pontage (66, 68 ; 78, 78) à chacune des deux demi-masses (64, 64),
ou
c3) l'assemblage des deux demi-masses (64, 64) par des formes respectives conjuguées complémentaires emboitables (84, 86) ; et
c4) la solidarisation définitive entre elles des deux demi-masses (64, 64) par les formes respectives conjuguées complémentaires emboitables (84, 86),
et dans lequel la lame PZT (22) comprend un orifice (70 ; 82, 82) et/ou une encoche (72 ;88, 88) aptes à être traversés :
à l'étape c1), par l'au moins une pièce transversale de pontage rapportée (66, 68 ; 78, 78)
ou, respectivement,
à l'étape c4), par un bossage (84) des formes conjuguées complémentaires (84, 86) des deux demi-masses.

6. Le procédé de la revendication 5,
dans lequel l'étape c4) comprend le soudage l'une à l'autre des deux formes respectives conjuguées complémentaires emboitables (84, 86).

7. Le procédé de la revendication 5,
dans lequel, les deux demi-masses (64, 64) comprenant lesdites formes respectives conjuguées complémentaires emboitables (84, 86), l'étape c4) comprend :
c4.1) la déformation en température de l'une des deux demi-masses ;
c4.2) l'emboitement des deux demi-masses l'une avec l'autre ; et
c4.3) le retour à température ambiante, pour assujettir entre elles par jeu négatif les deux demi-masses emboitées.

8. Un module de récupération d'énergie, PEH, comprenant :
- un tube enveloppe (50) allongé ;
- logé à l'intérieur du tube (50), un ensemble pendulaire (22, 26) selon l'une des revendications 1 à 4.

9. Un dispositif autonome logeant, dans un corps de dispositif :
- un ensemble électronique (28-38) ;
- un module PEH (40) selon la revendication 8, produisant en sortie un signal électrique ;
- un circuit de gestion d'alimentation (42), apte à redresser et réguler le signal électrique produit par le module PEH pour délivrer en sortie une tension ou un courant continus d'alimentation stabilisés ; et
- un organe de stockage d'énergie (44) pour l'alimentation de l'ensemble électronique,
dans lequel ladite tension ou ledit courant continus stabilisés délivrés par le circuit de gestion d'alimentation servent à l'alimentation de l'ensemble électronique et/ou à la recharge de l'organe de stockage d'énergie du dispositif autonome.

10. Le dispositif autonome de la revendication 9,
dans lequel le dispositif autonome est un dispositif médical actif de type capsule autonome implantable (10) comprenant un corps de capsule (12) avec un élément d'ancrage (16) à une paroi d'un organe d'un patient,
et dans lequel les sollicitations externes auxquelles est soumis l'ensemble pendulaire (22, 26) du module PEH sont des sollicitations appliquées au corps de capsule (12) sous l'effet de mouvements de ladite paroi et/ou de variations de débit d'un flux dans le milieu environnant.

## Patentansprüche

1. Eine Pendeleinheit für ein Energierückgewinnungsmodul, PEH, wobei die Pendeleinheit Folgendes umfasst:
- ein Längsorgan eines piezoelektrischen Wandlers (PZT), wobei sich das PZT-Längsorgan (22) in axialer Richtung erstreckt zwischen einem proximalen Ende, das in ein Spannelement (24) eingespannt ist, und einem freien distalen Ende, das elastisch biegeverformbar ist; und
- eine inertiale Masse (26), die an dem freien distalen Ende des PZT-Längsorgans (22) gelagert ist und in Querrichtung beweglich ist, wobei die inertiale Masse (26) zwei Halbmassen (64, 64) umfasst, die auf beiden Seiten des PZT-Längsorgans (22) an dem freien Ende von diesem angeordnet sind,
wobei die Pendeleinheit dazu eingerichtet ist, eine mechanische Energie, die von Schwingungen der Pendeleinheit unter der Einwirkung von äußeren Belastungen, die das Modul erfährt, erzeugt wird, in ein oszillierendes elektrisches Signal umzuwandeln, das von Oberflächenelektroden des PZT-Längsorgans (22) empfangen wird, wobei die Pendeleinheit eine mechanische Verbindung der zwei Halbmassen (64, 64) auf beiden Seiten des PZT-Längsorgans (22) umfasst, wobei die mechanische Verbindung dazu eingerichtet ist, das PZT-Längsorgan (22) zwischen den zwei Halbmassen (64, 64) einzuklemmen, um die inertiale Masse (26) an dem PZT-Längsorgan (22) zu befestigen, wobei die Montage frei von Klebstoff zwischen den Halbmassen (64, 64) und dem PZT-Längsorgan (22) ist,
**dadurch gekennzeichnet, dass** die mechanische Verbindung der zwei Halbmassen (64, 64) auf beiden Seiten des PZT-Längsorgans (22) Folgendes umfasst:
wenigstens ein angebrachtes quergerichtetes Überbrückungsteil (66, 68; 78, 78), das an jede der zwei Halbmassen (64, 64), und/oder
der jeweiligen ineinander steckbaren komplementären einander zugeordneten Formen (84, 86) der zwei Halbmassen (64, 64) festgeschweißt ist,
**und dadurch, dass** das PZT-Längsorgan (22) eine Öffnung (70; 82, 82), und/oder eine Einkerbung (72;88, 88) umfasst, die dazu eingerichtet sind, durchquert zu werden:
von dem wenigstens einen angebrachten quergerichteten Überbrückungsteil (66, 68; 78, 78) bzw.
von einem Höcker (84) der ineinander steckbaren komplementären einander zugeordneten Formen (84, 86) .

2. Die Pendeleinheit nach Anspruch 1, wobei das wenigstens eine angebrachte quer gerichtete Überbrückungsteil (66, 68; 78, 78) an dem distalen und/oder proximalen Ende der zwei Halbmassen (64, 64) angeordnet ist.

3. Die Pendeleinheit nach Anspruch 1, wobei das wenigstens eine angebrachte quergerichtete Überbrückungsteil (66, 68; 78, 78) in einer mittigen Zone der zwei Halbmassen (64, 64) angeordnet ist.

4. Die Pendeleinheit nach Anspruch 1, ferner umfassend wenigstens einen Schweißpunkt zwischen den jeweiligen ineinander steckbaren komplementären einander zugeordneten Formen (84, 86).

5. Ein Verfahren zum Montieren einer Pendeleinheit für ein Energierückgewinnungsmodul (PEH), wobei die Pendeleinheit ein Längsorgan (22) eines piezoelektrischen Wandlers (PZT), das elastisch biegeverformbar ist, und eine inertiale Masse (26), die an dem freien distalen Ende des PZT-Längsorgans (22) gelagert ist und in Querrichtung beweglich ist, umfasst,
wobei das Verfahren die folgenden Schritte umfasst:
a) Erhalten eines PZT-Längsorgans (22);
b) Anordnen, an dem freien distalen Ende des PZT-Längsorgans (22), von zwei Halbmassen (64, 64), die die inertiale Masse (26) bilden, auf beiden Seiten des PZT-Längsorgans (22); und
c) Erzeugen einer mechanischen Verbindung der zwei Halbmassen (64, 64) auf beiden Seiten des PZT-Längsorgans (22) umfasst, ohne Zufuhr von Klebstoff zwischen dem PZT-Längsorgan (22) und den zwei Halbmassen (64, 64), wobei die mechanische Verbindung dazu eingerichtet ist, das PZT-Längsorgan (22) zwischen den zwei Halbmassen (64, 64) einzuklemmen, um die inertiale Masse (26) an dem PZT-Längsorgan (22) zu befestigen,
wobei in dem Verfahren Schritt c) Folgendes umfasst:
c1) das Anordnen wenigstens eines quergerichteten Überbrückungsteils (66, 68; 78, 78) an dem distalen und/oder proximalen Ende der zwei Halbmassen (64, 64); und
c2) das Festschweißen des wenigstens einen quergerichteten Überbrückungsteils (66, 68; 78, 78) an jeder der zwei Halbmassen (64, 64),
oder
c3) das Montieren der zwei Halbmassen (64, 64) durch jeweilige ineinander steckbare komplementäre einander zugeordnete Formen (84, 86); und
c4) das endgültige feste Verbinden der zwei Halbmassen (64, 64) miteinander durch die jeweiligen ineinander steckbaren komplementären einander zugeordneten Formen (84, 86),
und wobei das PZT-Längsorgan (22) eine Öffnung (70; 82, 82) und/oder eine Einkerbung (72;88, 88) umfasst, die dazu eingerichtet sind, durchquert zu werden:
in Schritt c1), von dem wenigstens einen angebrachten quergerichteten Überbrückungsteil (66, 68; 78, 78)
bzw.
in Schritt c4), von einem Höcker (84) der komplementären einander zugeordneten Formen (84, 86) der zwei Halbmassen.

6. Das Verfahren nach Anspruch 5,
wobei Schritt c4) das Festschweißen der zwei jeweiligen ineinander steckbaren komplementären einander zugeordneten Formen (84, 86) aneinander umfasst.

7. Das Verfahren nach Anspruch 5,
wobei, wenn die zwei Halbmassen (64, 64) die jeweiligen ineinander steckbaren komplementären einander zugeordneten Formen (84, 86) umfassen, Schritt c4) Folgendes umfasst:
c4.1) das temperaturbedingte Verformen einer der zwei Halbmassen;
c4.2) das Ineinanderstecken der zwei Halbmassen ineinander; und
c4.3) das Wiederaufumgebungstemperaturbringen, um die zwei einander gesteckten Halbmassen durch negatives Spiel aneinander zu befestigen.

8. Ein Energierückgewinnungsmodul (PEH), umfassend:
- ein langgezogenes Mantelrohr (50);
- im Inneren des Rohrs (50) aufgenommen, eine Pendeleinheit (22, 26) nach einem der Ansprüche 1 à 4.

9. Eine autonome Vorrichtung, die in einem Vorrichtungskörper Folgendes aufnimmt:
- eine elektronische Anordnung (28-38);
- ein PEH-Modul (40) nach Anspruch 8, das ausgangsseitig ein elektrisches Signal erzeugt;
- eine Versorgungsverwaltungsschaltung (42), die dafür eingerichtet ist, das von dem PEH-Modul erzeugte Signal gleichzurichten und zu regeln, um ausgangsseitig eine stabilisierte Vorsorgungsgleichspannung oder einen stabilisierten Versorgungsgleichstrom bereitzustellen; und
- ein Energiespeicherorgan (44) für die Versorgung der elektronischen Anordnung,
wobei die stabilisierte Gleichspannung oder der stabilisierte Gleichstrom, die/der von der Versorgungsverwaltungsschaltung bereitgestellt werden, der Versorgung der elektronischen Anordnung und/oder der Aufladung des Energiespeicherorgans der autonomen Vorrichtung dienen.

10. Die autonome Vorrichtung nach Anspruch 9, wobei die autonome Vorrichtung eine aktive medizinische Vorrichtung des Typs implantierbare autonome Kapsel (10) ist, die einen Kapselkörper (12) mit einem Element (16) zur Verankerung an einer Wand eines Organs eines Patienten umfasst, und wobei die äußeren Belastungen, die die Pendeleinheit (22, 26) des PEH-Moduls erfährt, Belastungen sind, die auf den Kapselkörper (12) unter der Einwirkung von Bewegungen der Wand und/oder von Veränderungen einer Blutflussrate im umgebenden Medium aufgebracht werden.

## Claims

1. A pendular unit for an energy harvesting module, PEH, the pendular unit comprising:
- a piezoelectric, PZT, transducer beam, the PZT beam (22) extending axially between a proximal end, clamped into a clamping part (24), and a free distal end that is elastically deformable in bending; and
- an inertial mass (26) , that is mounted at the free distal end of the PZT beam (22) and mobile in a transverse direction, the inertial mass (26) comprising two half-masses (64, 64) arranged on either side of the PZT beam (22) at the free distal end of the PZT beam (22),
wherein the pendular unit is adapted to convert a mechanical energy produced by oscillations of the pendular unit under the effect of external stresses undergone by the module into an oscillating electrical signal collected by surface electrodes of the PZT beam (22),
wherein the pendular unit comprises a mechanical connection of the two half-masses (64, 64) to each other on either side of the PZT beam (22), the mechanical connection being adapted to clamp the PZT beam (22) between the two half-masses (64, 64) to secure the inertial mass (26) to the PZT beam (22), the assembly being devoid of glue between the half-masses (64, 64) and the PZT beam (22),
**characterized in that** the mechanical connection of the two half-masses (64, 64) to each other on either side of the PZT beam (22) comprises:
at least one added transverse bridging part (66, 68; 78, 78), welded to each of the two half-masses (64, 64), and/or
respective complementary and conjugated interlocking shapes (84, 86) of the two half-masses (64, 64),
**and in that** the PZT beam (22) comprises an orifice (70; 82, 82) and/or a notch (72; 88, 88) adapted to be passed through:
by the at least one added transverse bridging part (66, 68; 78, 78) or, respectively,
by a protrusion (84) of the complementary and conjugated interlocking shapes (84, 86).

2. The pendular unit of claim 1, wherein the at least one added transverse bridging part (66, 68) is arranged at the distal and/or proximal end of the two half-masses (64, 64).

3. The pendular unit of claim 1, wherein the at least one added transverse bridging part (78, 78) is arranged in a central area of the two half-masses (64, 64).

4. The pendular unit of claim 1, further comprising at least one welding point between the respective complementary and conjugated interlocking shapes (84, 86).

5. A method for assembling a pendular unit for an energy harvesting module, PEH, the pendular unit comprising a piezoelectric, PZT, transducer beam (22) that is elastically deformable in bending, and an inertial mass (26), that is mounted at the free distal end of the PZT beam (22) and mobile in a transverse direction,
the method comprising the following steps:
a) obtaining a PZT beam (22);
b) positioning at the free distal end of the PZT beam (22) two half-masses (64, 64) forming the inertial mass (26), on either side of the PZT beam (22); and
c) creating a mechanical connection of the two half-masses (64, 64) to each other on either side of the PZT beam (22), without addition of glue between the PZT beam (22) and the half-masses (64, 64), the mechanical connection being adapted to clamp the PZT beam (22) between the two half-masses (64, 64) to secure the inertial mass (26) to the PZT beam (22),
wherein method step c) comprises:
c1) positioning at least one transverse bridging part (66, 68; 78, 78) at the distal and/or proximal end of the two half-masses (64, 64); and
c2) welding the at least one transverse bridging part (66, 68; 78, 78) to each of the two half-masses (64, 64),
or:
c3) assembling the two half-masses (64, 64) by respective complementary and conjugated interlocking shapes (84, 86); and
c4) permanently securing the two half-masses (64, 64) to each other using the respective complementary and conjugated interlocking shapes (84, 86),
and wherein the PZT beam (22) comprises an orifice (70; 82, 82) and/or a notch (72; 88, 88) adapted to be passed through:
- at step c1), by the at least one added transverse bridging part (66, 68; 78, 78),
or, respectively,
- at step c4), by a protrusion (84) of the complementary and conjugated shapes (84, 86) of the two half-masses.

6. The method of claim 5,
wherein step c4) comprises the welding to each other of the two respective complementary and conjugated interlocking shapes (84, 86).

7. The method of claim 5,
wherein, the two half-masses (64, 64) comprising said respective complementary and conjugated interlocking shapes (84, 86), step c4) comprises:
c4.1) heat deforming one of the two half-masses;
c4.2) interlocking the two half-masses into each other; and
c4.3) getting back to room temperature, to secure the two interlocked half-masses together by a negative clearance.

8. An energy harvesting module, PEH, comprising:
- an elongated envelope tube (50);
- arranged inside the tube (50), a pendular unit (22, 26) according to any one of claims 1 to 4.

9. An autonomous device in which are arranged, within a device body:
- an electronic unit (28-38);
- a PEH module (40) according to claim 8, outputting an electric signal;
- a power management circuit (42), adapted to rectify and regulate the electric signal produced by the PEH module, to output a stabilized direct power voltage or current; and
- an energy storage component (44) for powering the electronic unit,
wherein said stabilized direct voltage or current provided by the power management circuit is used to power the electronic unit and/or to charge the energy storage component of the autonomous device.

10. The autonomous device of claim 9,
wherein the autonomous device is an active medical device of the implantable autonomous capsule type (10) comprising a capsule body (12) with an element (16) for its anchoring to a wall of a patient's organ,
and wherein the external stresses to which is subjected the pendular unit (22, 26) of the PEH module are stresses applied to the capsule body (12) under the effect of movements of said wall and/or flow rate variations of a flow in the surrounding environment.
